(19) Europäisches Patentamt
European Patent Office
Office européen des brevets

(11) Publication number: 0 270 026 A2

(12) # EUROPEAN PATENT APPLICATION

(21) Application number: 87117569.1

(51) Int. Cl.[4]: A61K 31/44

(22) Date of filing: 27.11.87

(30) Priority: 29.11.86 ZA 864001

(43) Date of publication of application: 08.06.88 Bulletin 88/23

(84) Designated Contracting States: AT BE CH DE ES FR GB GR IT LI NL SE

(71) Applicant: VESTA MEDICINES (PROPRIETARY) LIMITED
Holpro House Snell Street
Micor Johannesburg(ZA)

(72) Inventor: Serfontein, Willem Jacob
82 Ridgewater Road Lynnwood Manor
Pretoria(ZA)

(74) Representative: Vossius & Partner
Siebertstrasse 4 P.O. Box 86 07 67
D-8000 München 86(DE)

(54) **Pharmaceutical preparation comprising vitamin B6.**

(57) A pharmaceutical composition comprising as an ingredient and source of vitamin B6 pyridoxal (PL) or a physiologically compatible acid addition salt or complex of PL which in vivo rapidly release PL. Preferred further ingredients include a physiologically compatible antioxidant, e.g. ascorbic acid, glutamate, riboflavine and physiologically compatible salts of zinc and magnesium. The composition is used in humans and vetterinary medicine for the treatment or prophylaxis of one or more of the following:

(i) physiological shock
(ii) myocardial infarction
(iii) clinical conditions associated with elevated blood biogenic polyamine levels and depressed blood B6 activity
(iv) depressed blood B6 activity associated with the treatment of diseases or clinical conditions involving the use of B6-antagonistic drugs which cause or contribute to cellular B6 depletion and/or polyamine elevation. Preferred forms of administration are parenteral, e.g. by infusion.

EP 0 270 026 A2

## Pharmaceutical Preparation Comprising Vitamin B6

The present invention relates to a pharmaceutical composition comprising as an active ingredient a source of vitamin B6 and optionally a compatible extender, carrier or excipient and the uses of such composition and ingredient.

More particularly the invention relates to pharmaceutical substances and compositions for prophylaxis against or for counteracting vitamin B6 deficiences in blood plasma and in living cells, or for rapidly elevating such depressed levels, and new uses of certain B6 vitamers.

The vital role of vitamin B6 (hereinafter abbreviated to B6) in health and disease has been extensively researched over the past two decades. It is now realised that many serious diseases and clinical conditions are associated with reduced blood and cellular B6 activity. However, until now many of the physiological interrelationships in animals and humans have not been known or understood. In particular there has been confusion as to whether observations relating to B6 and B6 vitamer deficiencies were results of clinical conditions or whether these deficiencies were causally related with the clinical conditions. The was due inter alia to a disregard of certain of the pharmacokinetics involved, and of the role of different B6 vitamers. Prior to the present invention certain of these vitamers had never been determined systematically, and indeed no suitable methods had existed for such systematic determinations.

The vitamers PN, PL and PM and their phosphorylated forms PNP, and PMP were known. It has been proposed that plasma PL is derived from the liver and by hydrolysis of PLP before being oxidised to 4-pyridoxic acid (4-PA) and excreted. It has also been reported that PL is a transport form taken up by tissues with a low PNP content (Lumeng et al, "Vitamin B6 : its role in health and disease", 1985, Alan R Liss, p.35-54). These facts were interpreted as part of the mechanism involved in the metabolic pathway of PN. However, no clinical significance was attached to this.

The accepted and, to date, the only commercially available form of B6 in pharmaceutical preparations has been pyridoxine (PN). However, the conventional manners of administration of PN frequently do not lead to the desired alleviation of B6 deficiencies in blood plasma and in living cells, especially in critically ill patients. the consequences of such deficiencies can be most severe. Neither the extent of these consequences nor the causes thereof had been appreciated in the past, or adequately so. As will be explained further below, these B6 deficiencies are on the one hand caused or aggravated by a variety of physiological factors. They are in turn themselves the cause of a downbreak of vital physiological functions, which is often fatal. Many important drugs used in the treatment of diseases, whilst combatting the primary symptoms and/or causes of such diseases, have now been identified as severe contributors to B6 deficiencies which have unwittingly aggravated this downbreak of physiological functions. The administration of PN at best achieves partial and relatively slow alleviation of the B6 deficiency, and often not at all, particularly in the severely ill patient. The problem cannot be overcome by increasing the dosage of PN for reasons now established for the first time by us and explained in greater detail below.

There accordingly exists an unsatisfied need for a composition for use in human or animal therapy or prophylaxis capable of making available B6 effectively and at a required rate, to compensate rapidly for or to pre-empt excessive cell depletion of B6 during pathological states.

The present invention is base on the surprising finding that substantially improved and more rapid alleviation of B6 deficiency can be attained by the administration, in any suitable manner, but preferably parenterally, more particularly intravenously and most effectively by infusion, of pyridoxal (PL), either PL as such or in the form of a physiologically compatible acid addition salt or complex of PL which in vivo rapidly releases PL.

Pharmaceutical compositions comprising B6 in the form of PL have not been proposed or been available previously. In fact, there has been a distinct prejudice in the art against the administration of B6 in any form other than that of PN. That prejudice was based not only on cost but also on the relative stability and long shelf-life of PN. In vitro PL is far less stable. The art took no or insufficient cognisance of the fact that PN has an extremely short half-life in blood in vivo, that it is readily excreted and/or converted into physiologically unavailable compounds, and that PN must pass through a chain of biochemical reactions before it can enter the cells and finally end up there as required in the form of active vitamin B6, i.e. pyridoxal phosphate (PLP) and that this chain of reactions can be compromised at various stages.

Various facts are known, mostly in isolation, concerning B6, its occurrence and biochemistry. However, this knowledge has not been properly correlated, nor could the conclusions be drawn on which the present invention is based.

The present invention is based on our own, new experimental findings, including the pharmacokinetics of B6 vitamers in humans, a new interpretation of aspects of the biochemistry of B6 made possible by

these experimental findings, as well as new concepts based on the results of our experiments throwing new light on various biochemical observations which previously were not understood or coorelated. This has been made possible by our development for the first time of a practical method of determining PL in body fluids in the clinical situation.

According to a first aspect of the invention there is provided a pharmaceutical composition as set out in the opening paragraph, comprising the feature that it includes as such ingredient pyridoxal (PL) or a physiologically compatible acid addition salt or complex of PL which in vivo rapidly releases PL. Preferably the ingredient is PL or acid addition salt of PL.

According to certain embodiments, the composition comprises PL or a physiologically compatible acid addition salt or complex of PL which in vivo rapidly releases PL, or a mixture composed of two or more of these as the only source or sources of vitamin B6.

To protect against aerial oxidation, the composition (whether provided in solution form or as a solid, e.g. a powder) preferably contains an antioxidant, for example ascorbic acid (or a physiologically compatible salt thereof), the latter, for example, in a concentration of from 1 to 5000 mg per litre, preferably 50 - 500 mg per litre, e.g. 100 mg per litre, the concentrations given referring to composition in the form of a parenteral solution, e.g. an infusion solution or to the required antioxidant content of a composition provided in the form of a powder to yield the stated concentration in an infusion solution, prepared therefrom. Even near the lower limit, ascorbic acid is effective as an antioxidant. However, in the higher concentrations, ascorbic acid also becomes effective physiologically as a source of vitamin C where is often desirable, e.g. in infusion solutions.

Although PL is readily resorbed when administered for example per os, preferred embodiments, particularly for emergency use, are in a form adapted for or readily adaptable for parenteral administration, preferably intravenous administration and in particular in a form adapted for or readily adapted for intravenous infusion. The latter embodiments may contain the conventional ingredients of an infusion solution, for example electrolytes, glucose and/or conventional extenders, excipients and the like. Since B6 in the form of PL in solution has a limited shelf-life, the composition may alternatively be provided in the form of a dry powder, preferably vacuum-packed in dark-coloured ampoules, the dry powder containing all the required additives to permit immediate use of the composition as an infusion solution after dissolution in, and suitable dilution to the required concentration with, sterile water. Alternatively, the powdered form of the pharmaceutical composition may be adapted such that it may be dissolved in any other standard infusion solution used under these circumstances. As a further alternative the composition in powder form may be separately made up with sterile water and mixed with any other standard infusion solution before use, or said solution of the composition may be made up separately and administered together with a standard infusion solution after mixing in the feed tube of the infusion apparatus according to known art.

Preferred embodiments of the composition comprise as a further ingredient or ingredients one or more of the substances: glutamate, riboflavine, and physiologically compatible salts of zinc and magnesium. For example, these one or more substances are present in amounts to produce the following concentrations in an injection or infusion solution:

glutamate : from 0 to 500 mg/litre, preferably 10 to 200 mg/litre, e.g. 20 to 100 mg/litre
riboflavine : from 0 to 100 mg/litre, preferably 5 to 15 mg/litre, e.g. 10 mg/litre
zinc : from 0 to 200 mg/litre of $ZnCl_2$ or the molar equivalent of any other physiologically compatible zinc salt, preferably 5 to 15 mg/l, e.g. 10 mg/litre
magnesium : from 0 to 5 000 mg/litre of $MgCl_2$ or the molar equivalent of any other physiologically compatible magnesium salt, preferably at least 50 mg/litre, more specifically 500 to 2000 mg/litre, e.g. 800 mg/litre of $MgCl_2$,

and at least one of these substances is present in a finite, effective amount. It should be understood that these concentrations apply equally to a composition provided in the form of an infusion solution and to the additive content of a composition provided in the form of a powder to yield the stated concentrations in an infusion solution prepared therefrom. Preferably all four substances are present in the composition.

Alternatively, the composition of the pharmaceutical composition, e.g. infusion solution, is such that the following quantities of ingredients are provided daily (per 70 kg body mass of the patient) :

glutamate : 1 - 2 g, riboflavine : 10 - 20 mg,
zinc : 5 - 15 mg (as Zn),
magnesium : 500 - 1000 mg (as Mg), and
pyridoxal : 100 - 1000 mg.

Riboflavine, zinc ions and magnesium ions are co-factors in the enzymatic conversion of PN into PL, and zinc ions and magnesium ions are also co-factors in the phosphorylation of the vitamers PN and PL. Therefore these substances augment the conversion of PN when administered as part of the composition or separately, e.g. with the diet, even in patients suffering from reduced liver function to make more PL available to the body, and thereby augment the administration of PL in accordance with the present invention. Furthermore, as explained later in this specification, efficient glycolysis during ischaemia, e.g. in a coronary heart patient, is not only dependent on functionally available intracellular PLP, the phosphorylated form of PL (which according to the invention is supplemented by intravenous administration of a PL-containing infusion solution), but the action of the PLP during the critical period following a myocardial infarction (MI) incident is assisted by the provision of supplementary glutamate. Similar benefits arise from glutamate supplementation in other cells.

The present invention is based inter alia on the recognition that the administration of PL, rather than PN, or a physiologically compatible acid addition salt or complex of PL which in vivo rapidly releases PL offers distinct advantages in the treatment or prophylaxis of one or more of the following:

(i) physiological shock

(ii) myocardial infarction

(iii) clinical conditions associated with elevated blood biogenic polyamine levels and depressed blood B6 activity.

(iv) depressed blood B6 activity associated with the treatment of diseases of clinical conditions involving the use of B6 antagonistic drugs which cause or contribute to cellular B6 depletion and/or polyamine elevation.

Therefore, according to a further aspect of the invention, the present invention provides a new use of PL or a physiologically compatible acid addition salt or complex of PL which in vivo rapidly releases PL in the preparation of a medicine for treatment or prophylaxis of one or more of the conditions listed in the preceding paragraph.

The invention also provides a new use of PL or a physiologically compatible acid addition salt or complex of PL which in vivo rapidly releases PL in the manufacture of a medicine in combination with instructions for applying the medicine in the treatment or prophylaxis of one or more of the aforesaid conditions. In general the instructions are in written or printed form, more particularly provided on or in a package containing the medicine.

preferably PL is administered or released at a dosage rate of from 0,1 mg to 2 mg per kg of body mass per hour, more preferably from 0,2 mg to 0,6 mg, e.g. 0,5 mg/kg/hr.

The invention is based on the realisation that the aforesaid conditions are associated with often severely reduced blood and cellular B6 depletion and that the administration of PL is substantially more effective than PN to counteract such depletion, being effective even in patients who do not respond to conventional administration of PN. A direct inverse correlation exists between the degree of B6 depletion of patients and their chances of survival. Medical and surgical complications such as shock, hepatic failure, infection and sepsis, wound dehiscence, incisional hernia, aminoglycoside induced renal failure and impaired skin and mucosal regeneration, hemato-poiesis, hormone production and wound repair; multi-organ dysfunction, respiratory distress and also severe depression are substantially more frequent in B6 deficient patients. These conditions are, inter alia, causally related to impairment of immune function, collagen and elastin cross-linking and polyamine synthesis and accumulation. Those patients who ended up with a plasma content of phosphorylated PL (i.e. PLP) of 20 nM or greater usually survived, and those with a final PLP content of 10 nM or less usually did not. Patients who were destined to die had a poor response to conventional B6 supplementation: their increment in plasma PLP with hyperalimentation and additional PN was generally less than 10 nM and occasionally less than 1 nM, even with 200 mg/day of PN. The probable reasons why the administration of PL can overcome or mitigate this problem will be explained further below in this specification. However, the correctness or otherwise of any explanations given mechanisms postulated is not to limit the scope of the invention or to be relevant to the inventive merits of the invention as claimed.

Physiological shock may be defined as a state in which there is widespread and serious reduction of tissue perfusion which, if prolonged, leads to generalised impairment of cellular function. An important reason for the impaired cellular function is the reduced supply of essential nutrients (vitamins, glucose, etc.) to the cells as a result of the reduced tissue perfusion, which may ultimately lead to cell death and the liberation of cellular contents including B6 antagonists such as amines and polyamines such as spermine and spermidine into the surrounding body fluids, which in turn affect the vital B6 status of the patient, inter alia due to the reaction of B6 with polyamines and to the enzyme inhibitory properties of polyamines.

There are many different causes and types of shock, including hypovolaemia, internal sequestration, cardiogenic shock, trauma, obstruction of blood flow, neuropathic shock, infection, anaphylaxis, anoxia and others, including the terminal stages of many diseases, all resulting in B6 depletion in plasma and cells, which is physiologically harmful.

The basic common physiological defect induced by shock in reduced venous return and decreased cardiac output, which eventually lead to reduced tissue perfusion and ultimately cellular damage and death.

A complex form of shock may result from infections, which is often associated with the release of endotoxin, the lipopolysaccharide moiety of bacterial cell walls. In addition, many of the drugs used for the treatment of infections cause a further lowering of the B6 status of the patient; thus inhibiting the cellular energy producing mechanisms, and therefore compounding the clinical consequences.

Myocardial disease leading to MI is to be considered a particularly severe and often fatal physiological shock situation wherein cells, in this particular instance myocardial cells, enter into a physiological shock condition and undergo a depletion, and experience a resultant physiologically harmful shortage of B6, in particular PLP and PL. Such depletion is due to increased cellular demand and to the action of liberated polyamines (PA) and other amines known to be B6 antagonists. This is reflected in the lowered serum B6 levels seen in coronary patients immediately after the incident. According to the invention the harmful effect of physiological shock on the cells - i.e. infarction - is counteracted, mitigated, prevented or limited by the administration of PL to meet this shortage.

MI and other diseases involving shock may eventually enter a terminal phase characterised by the multi-organ failure syndrome, often due to the synergistic effect of the polyamines spermine and spermidine acting in unison with iatrogenically adminstered B6 antagonistic drugs. This is due to complex formation with B6 vitamers and/or enzyme inhibition. In the case of spermine, spermidine and many B6 antagonistic drugs, these complexes involve the irreversible formation of aldamine rings and permanent loss of B6 activity.

Drugs which are antagonistic to B6, and the toxicity of which aggravates the B6 depletion in patients due to spermine and other amines and polyamines released by dying cells (whereby the toxicities are synergistically enhanced), include aminoglycosides (e.g. gentamycin), cycloserine, theophylline-type drugs (including theophylline itself and in particular aminophylline), 1-dopa, dopamine, penicillamine, digoxin, oral contraceptives, anti-depressants (iproniazid, malamid, marpian), amphetamines, MAO inhibitors (e.g. phenelzine), amitryptylline, anti-TB(INH), azaribine (psoriasis), anti-hypertensives (hydralazine).

The highest toxic synergism results from drugs carrying 1,2 or 1,3 diamine moieties (e.g. $-CHNH_2-CHNH_2-$), e.g. gentamycin, histamine, resulting in the irreversible formation of aldamine rings.

Some of these drugs are normally given to patients suffering from severe diseases. However, although the drug may combat effectively the disease itself, there is an enhanced toxic effect and B6-lowering effect when given to severely ill patients with elevated polyamine levels. Death may result. As an example: gentamycin is routinely given to patients with severe gramnegative infections. The terminal phase is characterised by the multi-organ failure syndrome, often due to the synergistic effect of the polyamines spermine and spermidine acting in unison with iatrogenically administered B6 antagonistic drugs, such as gentamycin.

The administration of PN is often ineffective to counteract the B6 depletion. The present invention teaches to administer PL instead, preferably intravenously, to provide a rapid supply of active B6 in plasma and cells.

B6 occurs in three primary forms, known by their trivial names: pyridoxine (PN, formula I), pyridoxal (PL, formula II) and pyridoxamine (PM, formula III), as well as the corresponding phosphorylated forms PNP, PLP and PMP. Of these, PN is the form used in pharmaceutical formulations, as well as that occurring in plants.

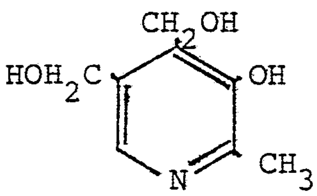 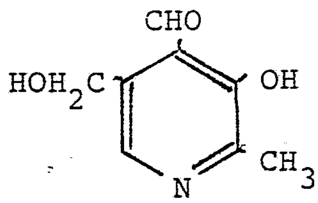 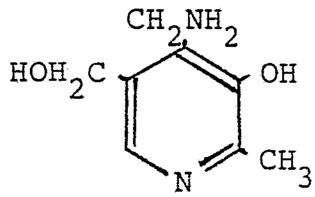

I PN

II PL

III PM

Inside living human and animal cells, PLP is the biologically active form of B6.

It is believed that the superiority of PL over other B6 vitamers, in particular PN, is based on the combination of principles and mechanisms postulated in what follows, which appears to fit biochemical and clinical observations:

PLP, i.e. the phosphorylated form of PL, is the active form of B6 inside cells as well as in the blood plasma. However, none of the phosphorylated B6 vitamers (PLP, PMP, PNP) is able to cross biological membranes (except those of the healthy liver), so that the intracellular levels of essential PLP depend upon the intra-cellular availability of PL or PN, which are the main precursors of PLP and which readily cross cell membranes.

PN as used in conventional B6 preparations must pass through a series of enzymatic reactions before it becomes available inside the various cells in its active form, PLP. Although PN, being non-phosphorylated, is capable of crossing biological membranes and entering into cells, it is useless inside the cell unless firstly it is converted (either before or after entering the cell) by the kinase enzyme into PNP, and secondly (and this must happen inside the respective cell) into PLP, by the action of the oxidase enzyme.

PL kinase is widely distributed in the body, including the myocardium. In contrast, the PN(PNP) oxidase system is confined to a few tissues and is either absent or present in very low concentrations in the heart and most other cells. It is present in relative abundance only in the liver, and even there only if the liver function is intact. This leads to the important conclusion that in tissues other than the healthy liver there is a long lag period for the synthesis of PLP after parenteral injection of PN, since conversion of PN into PLP takes place essentially only in the liver. If the liver function is impaired or absent (depressed liver function or liver perfusion), as may often happen in severely ill patients, particularly when multiple organ disfunction has set in, the conversion of PN into PL and PLP is compromised and PN-supplementation is rendered ineffective. It is a unique property of healthy liver tissue (as contrasted with other cells) that it is able to release to the plasma PLP bound to albumin.

Moreover, the plasma half-life of PN is man is only about 12 minutes. The administration of high doses of PN under these conditions is in fact counterproductive. The initial step of PN activation inside cells may either be kinase induced phosphorylation to PNP (followed by oxidase catalysed oxidation to PLP) or initially oxidase induced oxidation of PN to PL (followed by kinase catalysed phosphorylation to PLP). Both PN and PL therefore compete for the same kinase enzyme, and in the presence of excess PN, the phosphorylation of PL is compromised.

In addition it would appear that PN may inhibit the entry of PL into cells. PNP, once formed in any cells other than liver tissue, is metabolically trapped and is no longer available for conversion into PLP.

Administration of PLP is also substantially less effective than PL. PLP must first be de-phosphorylated in the plasma before it can enter the cells where B6 is needed. This involves a delay and presupposes the availability of the necessary enzymes (phosphotase) and the absence of factors inhibiting these enzymes. Moreover, PLP is substantially more prone to becoming protein (albumin) bound than PL.

This confirms the inventive concept that administration of PL is the only universally effective emergency procedure for restoring cellular B6 activity in serious disease conditions, whether or not this is accompanied by concomitant therapy with drugs that may be B6 antagonistic.

The clinical significance of PL was not previously recognised, inter alia because it was not appreciated that biological utilisation and activation of PN (by the liver) may be severely compromised in many acute disease condition, or that administration of PN under these circumstances may in fact be counterproductive in that high levels of PN not only suppress cellular PL uptake, but high intracellular PN levels compete with available PL for the common kinase enzyme system used to phosphorylate both PN and PL intracellularly.

The PLP plasma levels cannot be restored to normal in some critically ill patients even by giving large doses of PN, since those patients are no longer in a position to benefit metabolically from such treatment.

The invention teaches that in treating a coronary patient, in addition to the usual measures (streptokinase, beta-blockers, coronary vasodilators, etc.), the immediate intravenous administration (preferably by way of infusions) of a solution containing PL and electrolytes (either as such or in combination with glucose and other necessary co-factors, including riboflavine, zinc ions and magnesium ions) is an important and essential emergency step in limiting infarct size. Also, it is beneficial to administer glutamate.

Coronary patients have significantly depressed PLP levels during the 24 h period immediately following the MI incident, inter alia due to increased cellular demand during this period for PLP in the myocardium. This pathophysiological condition is considered to be due to oxygen deprivation of at least some of the tissues involved and to involve the following mechanisms:

The heart muscle is able to utilise different substrates as energy sources, including fatty acids, lactate and glucose. In the well-perfused and oxygenated heart, fatty acids are the prime source of energy, taking precedence over the process of glycolysis as energy source. However, in the absence of adequate supplies of oxygen following local myocardial ischaemia, energy production becomes dependent on the less efficient but rapidly mobilised process of glycolysis. The enzyme glycogen phosphorylase, which catalyses the hydrolysis of muscle glycogen to yield glucose required as energy source in the process of glycolysis, utilises PLP as co-enzyme. Efficient and rapid glycolysis therefore depends on the availability of adequate intracellular supplies of PLP, and this is achieved by infusion of PL.

During glycolysis, the oxidation of glyceraldehyde-3-phosphate to glycerate-1,3-diphosphate produces NADH (reduced form of nicotinamide adenine dinucleotide), which eventually leads to cellular depletion of NAD (nicotinamide adenine dinucleotide) unless the NADH produced is re-oxidised. Glucose oxidation via the process of glycolysis can therefore only continue if the NADH produced is removed in such a manner that NAD is regenerated. Since NADH is an inhibitor of the enzyme glyceraldehyde-3-phosphate dehydrogenase, feedback control by product inhibition plays a role in restricting glycolysis in ischaemic cells with impaired ability to re-oxidise NADH.

Normally in the well-oxygenated cell the electron transport chain situated in the mitochondria oxidatively removes the accumulated NADH. However, the mitochondrial membrane is impermeable to NADH, so that NADH produced in the cytosol cannot be removed in this manner. This problem can be circumvented in the myocardial cell by means of the malate-aspartate shuttle, which is capable of indirectly transporting electrons from NADH across the mitochondrial membrane, thus re-oxidising NADH to NAD. NADH in the cytoplasm is oxidised to NAD by donating electrons to reduce oxaloacetate to malate, the latter being readily transported across the mitochondrial membrane. Inside the mitochondrion, malate is re-oxidised to oxalo-acetate, with simultaneous reduction of NAD. The mitochondrial membrane is, however, impermeable to oxaloacetate, which would therefore accumulate unless effectively removed. This is achieved by means of a PLP dependent transamination of oxaloacetate to aspartate, the amino group in this exchange reaction being supplied by glutamate, a substance capable of freely crossing the mictochondrial membrane in either direction. The mitochondrial membrane is also freely permeable to the aspartate produced intra-mitochondrially in this manner, and it is therefore transported to the cytoplasm, where it is subsequently transaminated to oxaloacetate in a reaction which is again PLP dependent. Thus the cytoplasmic removal of NADH and confirmed effective glycolysis is dependent on the availability of adequate intracellular supplies of PLP, supplemented according to the invention by infusion of PL.

The aforegoing furthermore explains why, according to the invention, supplementation with glutamate is also of importance, since the malate-aspartate shuttle depends on the presence of glutamate in the ischaemic heart muscle cell (and indeed in other ischaemic cells as well). However, by supplying both PL and glutamate, the overall effect is more than would be expected from simple summation of the effects.

The foregoing also supports the teaching that PL administration, for example by means of a suitable infusion, may be used to reduce and control elevated polyamine (PA) levels in the blood associated with certain renal and other conditions in order to eliminate, prevent or pre-empt PA toxicity.

Yet another application of PL therapy or prophylaxis is when used as a means to increase the patient's B6 status before or even during major surgery or with any other critical condition, to improve the prognosis and outcome, especially in the debilitated, the elderly and those requiring massive procedures.

Since in many conditions, actual or incipient B6 deficiency (for example caused by increased PA levels arising from the condition or caused by medicaments used in the treatment) may not be suspected, the invention proposes the prophylactic inclusion of B6, partly or wholly in the form of PL, in essentially all infusion solutions and solutions used for parenteral administration. Furthermore, the inclusion of PL in infusion solutions may in general be used to decrease or control the "amine load" in the very ill patient.

No adverse effects of PL administration in experimental animals or humans are known.

In contrast to some other vitamins, the physiology and biochemistry relating to B6 differs relatively little between humans and animals. Accordingly, what has been described in the aforegoing in relation to humans is applicable also to veterinary medicine. Accordingly, the scope of the invention extends to veterinary medicine in relation to all aspects of the invention. The pharmaceutical compositions may be applied in the treatment of animal diseases and surgery substantially in a manner analogous to the treatment and prophylaxis described for humans.

A specific application would be in the context of game capture, and sedation and transportation of domestic or wild animals. For game capture the present invention teaches the inclusion of PL (optionally with other ingredients described above) in parenteral sedation an immobilising solutions, e.g. for darting syringes, as well as (and in particular) the inclusion of PL (and optional other ingredients) as part of or to be administered jointly with or in injectable antidote solutions as used in reversing the sedation and immobilisation after capture.

## EXAMPLES

Example 1 to 3 are suitably formulated as sterilised solutions having the compositions indicated (per 1000 ml of solution). In all cases due provision is to be made for osmolality requirements by addition of NaCl or by dilution.

The compositions given below may also be provided in powdered form (in proportions indicated) in sterile ampoules or other containers, for dissolution in sterile water and dilution to a prescribed volume immediately before use.

Example 1 : General solution for intravenous administration

| (a) | Range | Preferred range | Preferred |
|---|---|---|---|
| Pyridoxal.HCl* | 2 - 1000 mg | 20 - 100 mg | 50 mg |
| Glucose | 1 - 100 g | 20 - 80 mg | 50 g |
| $ZnCl_2$ | 1 - 1000 mg | 5 - 15 mg | 10 mg |
| Ascorbic acid | 1 - 5000 mg | 50 - 500 mg | 100 mg |

| (b) | Range | Preferred range | Preferred |
|---|---|---|---|
| Pyridoxal.HCl* | 2 - 500 mg | 20 - 300 mg | 100 mg |
| $ZnCl_2$ | 1 - 1000 mg | 5 - 15 mg | 10 mg |
| Ascorbic acid | 1 - 5000 mg | 50 500 mg | 100 mg |

*May be varied as desired to provide for an infusion rate per hour of PL of 5 to 500 mg (preferred 50 mg) in humans.

Preferred infusion rate (in both cases) : 50 to 150 ml/h (1,0 - 1,5 mg/kg/h).

Example 2 : Solution for the treatment of shock

a) Ringer's lactate solution NaCl = 6,0 g;
KCl = 400 mg;
Sodium lactate = 3,2 g;
$CaCl_2.H_2O$ = 270 mg;

Pyridoxal.HCl* = 1 to 1000 mg (preferred range: 20 to 200 mg; preferred: 100 mg.

Zinc chloride = 1 to 1000 mg (preferred range: 5 to 15 mg; preferred: 10 mg);
Ascorbic acid = 1 to 5000 mg (preferred range: 50 to 500 mg; preferred: 100 mg).

This solution contains electrolytes in mmol/l as follows: Na = 131, K = 5, Ca = 2, Cl = 111, lactate as $HCO_3$ = 29, and has millosmol/l of about 279 and pH of about 6,5

b) Electrolyte solution; NaCl = 6,0 g;
KCl = 300 mg;
NaHCO = 2,3 g;
$MgCl_2.6H_2O$ = 300 mg;
Pyridoxal.HCl * = 1 to 1000 mg (preferred range: 20 to 200 mg; preferred: 100 mg.

ZnCl 1 to 1000 mg (preferred range: 5 to 15 mg; preferred: 10 mg);
Ascorbic acid = 1 to 5000 mg (preferred range: 50 to 500 mg; preferred: 100 mg).

Rate of infusion 1,0 to 1,5 ml/kg/h.

This solution contains electrolytes in mmol/l as follows:
Na = 130, K = 4, Mg = 1,5, Cl = 109, $HCO_3$ = 28

and has milliosmol/l of 273 and pH of 7,4.

*May be varied as desired to provide for an infusion rate per hour of PL of 5 - 500 mg (preferred 50 mg).) in humans;

*May be varied as desired to 10 provide for an infusion arate per hour of PL of 5 - 500 mg (preferred 50 mg).) in humans;

Example 3 : Solution for intravenous infusion

| | Range | Preferred Range | Preferred |
|---|---|---|---|
| Pyridoxal.HCl* | 2 - 1000 mg | 20 - 100 mg | 50 mg |
| Pyridoxine.HCl | 1 - 1000 mg | 2 - 20 mg | 5 mg |
| Glucose | 1 - 100 g | 20 - 80 g | 50 g |
| Glutamate | 1 - 500 mg | 4 - 200 mg | 100 mg |
| $ZnCl_2$ | 1 - 500 mg | 5 - 15 mg | 10 mg |
| $MgCl_2$ | 5 - 5000 mg | 100 - 500 mg | 399 mg |
| Riboflavine | 1 - 100 mg | 5 - 20 mg | 10 mg |
| Ascorbic acid | 1 - 5000 mg | 50 - 500 mg | 100 mg |
| NaCl | (as required) | | |

Preferred rate of infusion : 1,0 to 1,5 ml/kg/h.

Example 4 : Per os (one daily dose)PL 10 - 500 mg, e.g. 200 mg
PN 10 - 500 mg, e.g. 200 mg
Riboflavine 1 - 100 mg, e.g. 10 mg
ZnCl 1 - 100 mg, e.g. 50 mg
MgCl 1 - 5000 mg, e.g. 2000 mg
Ascorbic acid 100 mg

Given as a solution or in the form of tablets or capsules, preferably in a slow release formulation.

Example 5 : SuppositoriesPL 10 - 500 mg, e.g. 200 mg
PN 10 - 500 mg, e.g. 200 mg
Riboflavine 1 - 100 mg, e.g. 10 mg
ZnCl 1 - 50 mg, e.g. 20 mg
MgCl 1 - 2000 mg, e.g. 1000 mg
Ascorbic acid 100 mg

These ingredients to be included with the usual excipients, carriers, etc. used in the formation of suppositories.

Example 6 : Immobilising/sedation compositions for game catching or transport

a) As an additive to the drug, or to be administered separately: (per 100 kg live mass)
PL: 10 - 2000 mg, e.g. 200 mg
PN: 10 - 100 mg, e.g. 50 mg

b) As an additive to the antidote solution, or to be administered separately (per 100 kg live mass)
PL: 10 - 2000 mg, e.g, 200 mg
PN: 10 - 500 mg, e.g. 100 mg
Riboflavine: 1 - 100 mg, e.g. 105 mg

Example 7 : Treatment of a patient during immediate post infarction period

Procedure A

In addition to the other drugs that may be indicated and that are used conventionally administered, the following solutions are administered by means of intravenous infusion:
Initial period (0 to 2 hours post-infarction):
Composition per litre: NaCl: 6,0 g, $NaHCO_3$ : 2,3 g, KCl: 300 mg, $MgCl_2.6H_2O$: 300 mg, Pyridoxal.HCl: 400 mg, rate of infusion: 80 to 120 mg/h (10 to 1,5 mg/kg/h).
Maintenance period: (2 to 12 h post-infarct):
Composition per litre: NaCl: 6,0 g, $NaHCO_3$ : 2,3 g, KCl: 300 mg, $MgCl_2.6H_2O$: 300 mg, Pyridoxal.HCl: 100 mg.
Rate of infusion: 80 - 120 ml/h (1 - 1,5 ml/kg/h)

Procedure B

Initial period (0 - 2 h post-infarction):
Composition per litre : Glucose: 50 g, Pyridoxal: 100 mg, Pyridoxine: 100 mg, Riboflavine: 5 mg, $ZnCl_2$: 10 mg.
Rate of infusion: 1 to 2 ml/kg/h.

Example 8 : Administration of pyridoxal containing infusion to critically ill patients

Composition per litre: NaCl: 6,0 g, $NaHCO_3$ : 2,3 g, KCl: 300 mg, Mg $Cl_2.6H_2O$: 300 mg, Pyridoxal.HCl: 400 mg

| | |
|---|---|
| Low rate of infusion: | 50 ml/h |
| Intermediary rate of infusion: | 80 ml/h |
| High rate of infusion: | 120 ml/h |

Example 9 : Protective effect of PL in experimentally induced septic shock in animals (rats)

An E. Coli suspension was prepared and serially diluted to the point where an injection of 0.3 ml caused approximately 80% mortality in rats after 12 h. This solution was used as the challenging solution in the following experiment:

Two groups of 6 male rats each were used. The animals were carefully selected so that the average mass in both groups was 210 g (191 - 219 g in the control group; 194 - 224 g in the experimental group). A solution containing 43,8 mg/ml of pyridoxal was prepared for i/v and i/p injection. 0,8 ml of this solution afforded 1,0 m mol/kg of PL when injected into a 210 g rat.

At time 0 h, the control received 0,3 ml saline and the experimental group 0,3 ml of the PL solution. After 1 h all animals in both groups were challenged with 0,3 ml i/v of the E. Coli suspension. Immediately thereafter, the control group received 0,5 ml saline and the experimental group 0,5 ml of the PL solution i/p. The animals were observed for a period of 12 h and the survivors counted.

The results are summarised in the following table. Each entry represents 1 animal, 12 h denoting that the particular animal survived for the full observation period of 12 h. Other entries denote mortalities at the times indicated, e.g. 2 h inspection (all animals were inspected at 1 h intervals and mortalities counted).

ResultsControl Group
1h, 1h, 1h, 1h, 1h, 4h

PL-supplemented Group
1h, 3h, 3h, 3h, 12h, 12h

The results indicate a statistically significant difference in survival rate.

Example 10 : Cellular uptake of PL

Fibroblast cell cultures were cultivated in the usual manner in a medium containing different concentrations of various B6 vitamers, and the intracellular PLP content was determined. The results indicated that intracellular PLP levels could be increased by increasing extracellular PN and/or PL concentrations, but that PL was more effective than PN in doing so. Extracellular PLP was not accumulated by the cells. This observation was confirmed by incubating the cells with radioactive PLP: very little radioactivity appeared in the intracellular fraction. When PN was used in the medium, intracellular PLP levels increased much more slowly (and in a time dependent manner) than when PL was used as extracellular source.

When the extracellular PL concentration was 60 nM, intracellular PLP levels reached a concentration of 12 nmol/g cell protein. When extracellular levels were increased, intracellular PLP levels increased correspondingly in an approximately linear fashion until a maximum value of 120 nmol/g cell protein was reached at an extracellular PL concentration of 600 nM. Further increases in intracellular PLP values indicated that cellular PL uptake is a saturable process. Moreover, when both PN and PL were included in the medium, intracellular PLP accumlation was decreased in comparison with PLP accumulation seen when PL alone (in equimolecular concentrations) was included in the medium. This effect was most noticeable in the higher concentration ranges and indicates that high concentrations of PN actually suppress intracellular PLP accumulation.

**Claims**

1. A pharmaceutical composition comprising as an active ingredient a source of vitamin B6 and optionally a compatible extender, carrier or excipient, characterised in that it includes as such ingredient pyridoxal (PL) or a physiologically compatible acid addition salt or complex of PL which in vivo rapidly releases PL.

2. A pharmaceutical composition as claimed in claim 1, characterised in that the ingredient is PL or an acid addition salt of PL.

3. A pharmaceutical composition as claimed in claim 1 or 2, characterised in that it comprises PL or a physiologically compatible acid addition salt or complex of PL which in vivo rapidly releases PL or a mixture composed of two or more of these as the only source or sources of vitamin B6.

4. A pharmaceutical composition as claimed in claim 1, 2 or 3, characterised in that it contains a physiologically compatible antioxidant.

5. A pharmaceutical composition as claimed in claim 4, characterised in that the antioxidant is ascorbic acid or a physiologically compatible salt of ascorbic acid.

6. A pharmaceutical composition as claimed in any one of claims 1, 2, 4 or 5, characterised in that it contains pyridoxine (PN) as a further source of vitamin B6.

7. A pharmaceutical composition as claimed in any one of claims 1 to 6, characterised in that it is in a form adapted for or readily adaptable for parenteral administration.

8. A pharmaceutical composition as claimed in claim 7, characterised in that it is in a form adapted for or readily adapted for intravenous infusion.

9. A pharmaceutical composition as claimed in claim 7 or 8, characterised in that it is provided as a ready to use dry powder adapted to dissolved in an injection or infusion medium.

10. composition as claimed in any one of claims 7 to 9, characterised in that it contains ascorbic acid or a compatible salt thereof in an amount to produce a concentration of from 1 to 5000 mg per litre of injection or infusion solution.

11. Composition as claimed in any one of the preceding claims comprising as a further ingredient or ingredients one or more of the substances: glutamate, riboflavine, physiologically compatible salts of zinc and magnesium.

12. A pharmaceutical composition as claimed in claim 11, characterised in that the one or more substances are present in amount to produce the following concentrations in an injection or infusion solution:

glutamate : from 0 - 500 mg/litre, preferably 10 to 200 mg/litre, e.g. 20 to 100 mg/litre

riboflavine : from 0 - 100 mg/litre, preferably 5 to 15 mg/litre, e.g. 10 mg/litre

zinc : from 0 - 200 mg/litre of $ZnCl_2$ or the molar equivalent of any other physiologically compatible zinc salt, preferably 5 to 15 mg/litre, e.g. 10 mg/litre

magnesium : from 0 to 5 000 mg/litre of $MgCl_2$ or the molar equivalent of any other physiologically compatible magnesium salt, preferably at least 5 mg/litre, more specifically 100 to 500 mg/litre, e.g. 300 mg/litre

and at least one of these substances is present in a finite, effective.

13. A composition as claimed in any one of claims 1 to 12 for use in the treatment or prophylaxis of physiological shock.

14. A pharmaceutical composition as claimed in claim 13 for use in the treatment or prophylaxis of myocardial infarction.

15. A composition as claimed in any one of claims 1 to 14 for use in the treatment or prophylaxis of clinical conditions associated with elevated blood biogenic polyamine levels and depressed blood B6 activity.

16. A composition as claimed in any one of claims 1 to 15 for use in the concomitant treatment or prophylaxis of depressed blood B6 activity associated with the treatment of diseases or clinical conditions involving the use of B6 antagonistic drugs which cause or contribute to cellular B6 depletion and/or polyamine elevation.

17. The use of PL or a physiologically compatible acid addition salt or complex of PL which in vivo rapidly releases PL in the preparation of a medicine for the treatment or prophylaxis of conditions resulting in or associated with a deficiency of physiologically active vitamin B6 in blood plasma and in living cells, in particular of one or more of the following diseases:

(i) physiological shock

(ii) myocardial infarction

(iii) clinical conditions associated with elevated blood biogenic polyamine levels and depressed blood B6 activity

(iv) depressed blood B6 activity associated with the treatment of diseases or clinical conditions involving the use of B6-antagonistic drugs which cause or contribute to cellular B6 depletion and/or polyamine elevation.

18. The use of PL or a physiologically compatible acid addition salt or complex of PL which in vivo rapidly releases PL in the manufacture of a medicine in combination with instructions for applying the medicine in the treatment or prophylaxis of one or more of the following:

(i) physiological shock

(ii) myocardial infarction

(iii) clinical conditions associated with elevated blood biogenic polyamine levels and depressed blood B6 activity

(iv) depressed blood B6 activity associated with the treatment of diseases or clinical conditions involving the use of B6-antagonistic drugs which cause or contribute to cellular B6 depletion and/or polyamine elevation.

19. The use as claimed in claim 18, wherein the instructions are in written or printed form.

20. The use as claimed in claim 19, wherein the instructions are provided on or in a package containing the medicine.

21. The use as claimed in any one of claims 17 to 20 in veterinary medicine.

22. The use as claimed in claim 21 in the context of game capture, sedation and transportation of domestic or wild animals.